# EUROPEAN PATENT APPLICATION

(11) **EP 2 740 495 A1**
(43) Date of publication of application: **11.06.2014**
(21) Application number: 12425195.0
(22) Date of filing: 07.12.2012
(51) Int. Cl.: A61L 2/18, A61L 2/24, B67B 3/00

(54) **Unit and method for sterilizing caps to be applied onto containers**

(71) Applicant: SIDEL S.p.A. con Socio Unico, Parma (IT)
(72) Inventor: Fusaro, Alessandro, 43100 Parma (IT)
(74) Representative: Di Sciuva, Michele

(57) **Abstract**

There is described a sterilizing unit (1) for sterilizing caps (2) to be applied onto respective containers (19), comprising: feeding means (4) for individually feeding caps (2) along a path (P); and a sterilizing station (5) arranged along path (P) and adapted to cause the interaction of a sterilizing agent with caps (2) so as to sterilize caps (2); sterilizing station (5) comprises a compartment (6) filled with a bath (7) of sterilizing agent and through which feeding means (4) individually feed caps (2).

## Description

The present invention relates to a sterilizing unit for sterilizing caps to be applied onto containers.

The present invention also relates to a method for sterilizing caps to be applied onto containers.

In particular, the above identified containers may be bottles or the like, which are filled with a liquid food product, e.g. fruit juice, tea or energy drinks.

In general, those containers are typically produced inside a production line essentially comprises:
- a pre-sterilizing unit for sterilizing a plurality of pre-forms;
   a blowing unit for forming the containers starting from respective pre-forms;
- a filling unit for filling the containers with the liquid food product;
- a capping unit for applying a plurality of caps onto respective containers; and
- a labelling unit for applying a plurality of labels onto respective containers.

The machine also comprises a housing which houses at least some of the above station, in particular the filling station and the capping station, and inside which there is a pressure greater than the environment value in order to prevent any contamination from the external environment.

Due to the fact that the containers are filled with a food product, it is necessary to sterilize the caps, before that the latter are applied to the respective containers.

A sterilizing unit is known, substantially comprising:
- a feeding system for conveying the caps along a sterilizing path; and
- a sterilizing station at which a sterilizing agent, e.g. hydrogen peroxide, interacts with the caps in order to sterilize those caps.

In greater detail, the feeding system comprises a hopper containing the caps to be sterilized, an elevator and a rail onto which caps moves long the sterilizing path.

Caps are moved onto the rail by a compressed airjet at a pressure greater than the environment pressure, e.g. 1,5 bar.

The sterilizing station substantially comprises a tunnel area inside which caps move along the rail from an inlet end a outlet end.

The tunnel is kept at a temperature of 95 °C

The sterilizing station comprises, proceeding from the inlet station to the outlet end, the following areas arranged inside the tunnel:
- a dosing area, inside which a flow of the sterilizing agent at high temperature, e.g. 130 °C, is directed towards the caps;
- a migration area, inside which the sterilizing agent is activated and actually carries out the sterilization of the caps; and
- a drying area, inside which an airflow is directed onto the caps, so as to cause the removal of the sterilizing agent.

The outlet end the sterilizing station is connected with the housing of the machine.

Furthermore, the sterilizing station comprises:
- a first and a second extractor arranged on the opposite sides of the dosing area; and
- a third extractor arranged at the end of the drying area.

Those extractors suck out from the tunnel the vapour of the sterilizing agent.

The effective position of the dosing area is therefore determined by the pressure field generated by the extractors into the tunnel.

In this respect, the Applicant has found that the pressure existing inside the tunnel is disturbed by the fact that a pressure greater than the environment pressure is generated in the tunnel in order to feed the caps and to dry to the caps and at the end of the tunnel.

As a result, the effective position of the dosing area is hardly controllable and strongly strongly relies to the skills of the technician.

Moreover, a potential incorrect effective position of the dosing area cannot be automatically detected by the operator.

A need is felt within the industry to obtain a sterilizing unit for sterilizing caps to be applied onto respective containers, which can precisely contain the dosing area in a given region, without relying onto the skills of the technician.

Finally, due to the fact that both the dosing are and the drying area are contained inside the same tunnel, the space available for the drying station is relatively small and, therefore, the drying action can be exerted for a relatively small time.

As a result, the airflow is directed at a relatively high temperature, e.g. about 100 °C, towards the caps in the drying area, thus generating the risk of partly melting the plastic material forming those caps.

A need is felt within the industry to reduce the risk that the caps are melted in the drying area.

It is therefore an object of the present invention to provide a sterilizing unit for sterilizing caps to be applied onto respective containers allowing to meet at least one of the aforementioned needs in a simple and cost-effective manner.

This object is achieved by a sterilizing unit for sterilizing caps to be applied onto respective containers, as claimed in claim 1.

The present invention also relates to method for sterilizing caps to be applied onto respective containers, as claimed in claim 11.

A preferred, non-limiting embodiment of the present invention will be described by way of example with reference to the accompanying drawings, in which:
- Figure 1 is a schematic view of a sterilizing unit, in accordance with the present invention; and
- Figure 2 shows a cap sterilized by the sterilizing unit of Figure 1 together with a corresponding container.

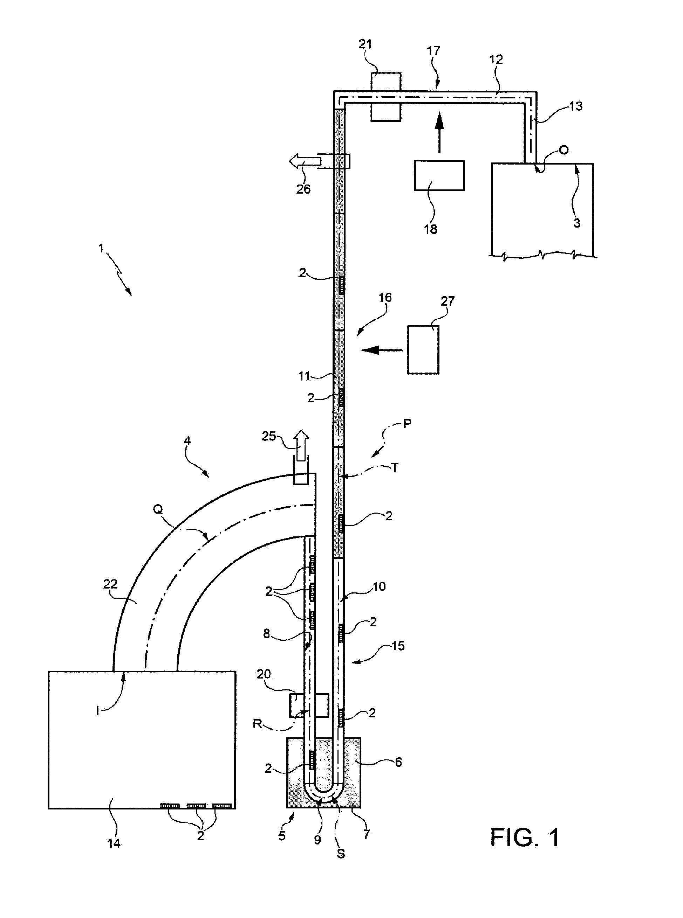

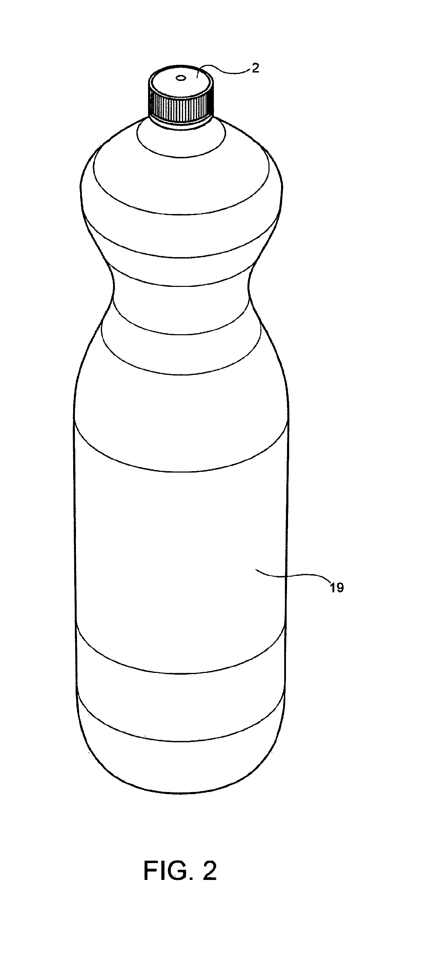

Number 1 in Figures 1 indicates as a whole a sterilizing unit for sterilizing caps 2 to be applied onto respective containers 19 (shown in Figure 2).

In particular, containers 19 are filled with a pourable, preferably liquid, food product, e.g. juice fruit, tea, energy drinks.

Sterilizing unit 1 is adapted to be incorporated in a not-shown machine for producing containers 19 filled with the food product.

The machine comprises, in particular,:
- a pre-sterilizing unit for sterilizing a plurality of pre-forms;
- a blowing unit for forming containers 19 from respective pre-forms;
- a filling unit for filling containers 19 with the food product;
- a capping unit for applying respective caps 2 to relative containers 19; and
- a labelling unit for applying a plurality of labels to respective containers 19.

The machine also comprise a housing 3 housing some of the above station, in particular the capping station.

The pressure inside housing 3 is kept at a pressure greater than environment value, 18 times the environment pressure, in order to prevent any contamination from the environment.

Sterilizing unit 1 substantially comprises:
- a conveying system 4 for individually conveying caps 2 from an inlet end I to an outlet end O along a path P;
- a sterilizing station 5 for sterilizing caps 2 advancing along path P by causing the interaction with a sterilizing agent.

In the embodiment shown, the sterilizing agent is hydrogen peroxide.

In detail, conveying system 4 comprises, proceeding from end I to end 0,:
- a hopper 14 containing caps 2;
- an elevator 22 for elevating caps 2 from hopper 14;
- a conduit 8 along which caps 2 fall down, under gravity action, from elevator 6;
- a wheel 9 (schematically shown in Figure 1) which receives caps 2 which have fallen from elevator 22 along conduit 8; and
- a conveyor 10 which receives caps 2 from wheel 9 and feeds caps 2 towards station O.

Elevator 22 is of the type shown in the International application WO-A-2012/001251 or WO-A-2012/001252. The content of WO-A-2012/001251 or WO-A-2012/001252 as regard to elevator 22 is incorporated by reference in the present application.

Conduit 8 is, in the embodiment shown, vertical.

Conveyor 10 comprises, in the embodiment shown, a vertical portion 11 starting from wheel 9, a horizontal portion 12 and a vertical portion 13 which defines outlet end O.

Conveyor 10 is, in the embodiment shown, a belt conveyor.

Path P comprises:
- an inclined stretch Q formed by elevator 22;
- a vertical stretch R formed by conduit 8;
- a U-shaped stretch S formed by wheel 9; and
- a stretch T formed by conveyor 10.

Advantageously, sterilizing station 5 comprises a compartment 6 filled with a bath 7 of the sterilizing agent and through which conveying system 4 individually feeds cap 2.

In particular, conveying system 4 feeds caps 2 inside bath 7 one after the other and one separate from the other. Furthermore, each cap 2 is not connected to other caps 2, when it passes through bath 7.

Sterilizing unit 1 also comprises:
- a migration station 15 arranged downstream of compartment 6 along path P and inside which the sterilizing agent is activated and, thus, sterilizes caps 2;
- a drying station 16 arranged downstream of migration station 15 along path P and inside which an airflow is directed by a plurality of nozzles 27 towards cap 2 in order to dry those caps 2; and
- a removal station 17 comprising a UV radiation source 18 which directs a UV radiation onto caps 2 in order to remove the sterilizing agent from those caps 2.

In the embodiment shown, migration station 15 is arranged along stretch T of path P.

Furthermore, the temperature inside migration station 15 ranges between 80-110 °C and is preferably about 95 °C.

In the embodiment shown, drying station 16 is arranged along stretch T of path P.

More precisely, migration station 15 and drying station 16 are arranged at portion 11 of conveyor 10.

Furthermore, nozzles 27 are configured for generating inside drying station 16 alternately an airflow at less than 100 °C, in particular 80°C, and an airflow at less than 80°C, in particular at 50°C.

Alternatively, nozzles 27 are configured for generating inside drying station 16 an airflow at only one temperature less than 100°C, in particular at 80°C.

In the embodiment shown, removal station 17 is arranged at portion 12 of conveyor 10 and along stretch T of path P.

Furthermore, in the embodiment shown UV radiation source 18 comprises one or more UV lamps.

Unit 1 also comprises:
- an orienting device 20 (only schematically shown Figure 1) arranged upstream of sterilizing station 5 and adapted to orient, one after the other, caps 2 in a first way before that caps 2 enter sterilizing station 5; and
- an orienting device 21 (only schematically shown in Figure 1) arranged downstream of sterilizing station 5 and adapted to orient, one after the other, caps 2 coming out from sterilizing station 5 in a second way.

In particular, the first way is the one required by sterilizing station 5 to ensure a deep penetration of sterilizing agent inside caps 2.

The second way is the one required by the operation of capping station.

Orienting device 20 is arranged, in the embodiment shown, along stretch R of path P and at conduit 8.

Orienting device 21 is arranged, in the embodiment shown, along stretch T of path P and at portion 12 of conveyor 10.

Wheel 9 is housed inside compartment 6.

Unit 1 also comprises an extractor 25 and an extractor 26 which suck out vapor of the sterilizing agent from unit 1.

In the embodiment shown, extractor 25 is arranged at the interface between elevator 22 and conduit 8, and, therefore, at interface between stretches Q, R of path P.

Extractor 26 is arranged downstream of drying station 16 proceeding along stretch T according to the advancing direction of caps 2 along path P.

More precisely, extractor 26 is arranged along portion 11 of conveyor 10.

In actual use, conveying system 4 individually feeds caps 2 along path P and through bath 7.

It is important to stress that conveying system 4 feeds each cap 2 separate from other caps 2.

In other words, each cap 2 is not connected to other caps 2.

In particular, caps 2 are normally housed inside hopper 14 and are raised by elevator 22 along stretch Q.

Then, caps 2 fall by gravity inside conduit 8 and along stretch R of path P.

Orienting device 20 individually orient caps 2 in the first way, as those caps 2 are travelling along conduit 8.

At this stage, caps 2 individually enter compartment 6 and individually pass through bath 7.

As a result, the sterilizing agent contained inside bath 7 contacts caps 2.

In particular, wheel 9 which is housed inside compartment 6 and bath 7 individually conveys caps 2 from conduit 8 to conveyor 10 and along stretch S.

Portion 11 of conveyor 10 individually conveys caps 2 at first in migration station 15 and then in drying station 16 along stretch T of path P.

Due to the fact that in migration station 15 the temperature is about 95°C, the sterilizing agent is activated in migration station 15 and therefore sterilizes caps 2.

In drying station 16, airflows at a temperature of about 80 degrees and at a temperature lower than 80 degrees are alternately directed onto caps 2, in order to dry the sterilizing agent from caps 2.

Alternatively, unit 1 is configured for generating inside drying station 16 an airflow at only one temperature less than 100°C, in particular at 80°C.

Subsequently, portion 12 of conveyor 10 individually conveys caps 2 towards orienting device 21 and removal station 17.

Orienting device 21 individually orient caps 2 in the second way, which is the one required by capping station of machine.

UV radiation source 18 directs the UV radiation onto caps 2 travelling into removal station 17.

In this way, the sterilizing agent is completely removed from caps 2.

Then, portion 13 of conveyor 10 individually conveys caps 2 towards outlet end O, whereat caps 2 are enters housing 3 at pressure higher than environment one.

Extractors 25, 26 are effective in sucking away the sterilizing agent vapor from unit 1

The advantages of unit 1 and of the method according to the present invention will be clear from the above description.

In particular, the sterilizing agent form bath 7 which is contained inside compartment 6.

Accordingly, the position of sterilizing agent is in no way affected by the pressure field existing inside sterilizing station 5.

In this way, contrary to the prior art discussed in the introductory part of the present description, there is no longer any problem in controlling the position of the sterilizing agent, which coincides with compartment 6.

As a result, the position of the sterilizing agent does not rely on the skills of the technician and the problem of detecting a potential incorrect effective position of the sterilizing agent substantially does not affect unit 1.

Moreover, the position of the sterilizing agent is in no way affected by the operative conditions of extractors 25, 26 and by the pressure existing in housing 3.

Furthermore, orienting devices 20, 21 are effective in individually orienting caps 2 respectively upstream of and downstream of sterilizing station 5.

Accordingly, orienting device 20 ensures that caps 2 enter sterilizing station 5 in the right position to ensure that the sterilizing agent fills the whole caps 2.

In the very same way, orienting device 21 ensure that caps 2 come out from sterilizing station 5 in the right position required by the capping unit.

Drying station 16 is defined by conveyor 10 which is different from wheel 9 which conveys caps 2 inside bath 7.

Accordingly, drying station 16 can be made enough long parallel to path P to ensure that the sterilizing agent can be dried by an airflow at less than 100°C and/or even by an airflow at a temperature lower than 80°C.

In this way, the risk that the caps 2 are melted by a long exposure to high temperature is dramatically reduced.

Finally, UV radiation source 18 is effective in removing all the residues of the sterilizing agent from caps 2 before that the latter applied onto respective containers 19.

Clearly, changes may be made to unit 1 and to the method as described and illustrated herein without, however, departing from the scope defined in the accompanying Claims.

## Claims

1. A sterilizing unit (1) for sterilizing caps (2) to be applied onto respective containers (19), comprising:
- feeding means (4) for individually feeding said caps (2) along a path (P); and
- a sterilizing station (5) arranged along said path (P) and adapted to cause the interaction of a sterilizing agent with said caps (2) so as to sterilize said caps (2);
**characterized in that** said sterilizing station (5) comprises a compartment (6) filled, in use, with a bath (7) of said sterilizing agent and through which said feeding means (4) individually feed said caps (2).

2. The sterilizing unit according to claim 1, **characterized by** comprising a first orienting station (20) arranged upstream of said sterilizing station (5) with reference to the advancing direction of said caps (2) along said path (P) and adapted to individually orient said caps (2) in a first way upstream of sterilizing station (5).

3. The sterilizing unit according to claim 1 or 2, **characterized by** comprising a second orienting station (21) arranged downstream of said sterilizing station (5) with reference to said advancing direction of said caps (2) and adapted to individually orient said caps (2) in a second way downstream of said sterilizing station (5).

4. The sterilizing unit of any one of the foregoing claims **characterized by** comprising a drying station (16) arranged downstream of said sterilizing station (5) with reference to said advancing direction;
said drying station (16) comprising means for directing (27) an airflow at a temperature lower than 80°C towards said caps (2).

5. The sterilizing unit of any one of the foregoing claims, **characterized by** comprising a removal station (17) arranged downstream of said drying station (16) with reference to said advancing direction and adapted to remove said sterilizing agent from said caps (2) ;
said removal station (17) comprising means for directing (18) a UV-radiation towards said caps (2).

6. The sterilizing unit of any one of the foregoing claims, **characterized by** comprising a first and a second extractor (25, 26) arranged on the opposite sides of said sterilizing station (5) along said path (P) ;
said first and second extractors (25, 26) being configured to allow the sterilizing agent vapour to escape said sterilizing station (5).

7. The sterilizing unit of any one of the foregoing claims, **characterized by** comprising an outlet station (O) operatively connected, in use, to an area at which the pressure is greater than pressure existing inside said sterilizing station.

8. The sterilizing unit according to any one of the foregoing claims, **characterized in that** said feeding means (4) comprise a first conveyor (9) housed inside said compartment (6).

9. The sterilizing unit according to claim 8, when depending on any one of claims 4 to 7, **characterized in that** said feeding means (4) comprises a second conveyor (10) housed outside said compartment (6) and which feeds, in use, said caps (2) at said drying station (16).

10. A machine for producing containers (19), comprising:
- a sterilizing unit (1) according to any one of the foregoing claims;
- at least one capping unit for applying said caps (2) onto respective containers (19); and
- a housing (3) defining said area at which the pressure is greater than the pressure existing inside said sterilizing station, and housing at least said capping unit.

11. A method for sterilizing caps (2) to be applied onto respective containers (19), comprising the steps of:
- individually feeding said caps (2) along a path (P); and
- sterilizing said caps (2) along said path (P) by causing the interaction of said caps (2) with a sterilizing agent;
**characterized in that** said step of feeding comprises the step of individually feeding said caps (2) through a compartment (6) filled, in use, with a bath (7) of said sterilizing agent.

12. The method of claim 11, **characterized by** comprising the steps of:
- orienting said caps (2) in a first way before said step of sterilizing; and/or
- orienting said caps (2) in a second way after said step of sterilizing.

13. The method of claim 11 or 12, **characterized by** comprising the steps of extracting the vapor of said sterilizing agent before and/or after said step of sterilizing.

14. The method of any one of claims 12 to 13, **characterized by** comprising the step of drying said caps (2) after said step of sterilizing; said step of drying comprising the step of directing an airflow at a temperature being lower than 80 °C towards said cap (2).

15. The method of any one of claims 11 to 14, **characterized in that** said step of feeding comprises the step of feeding said caps (2) after said steps of sterilizing and of drying at a first value of pressure greater than a second value of pressure at which said caps (2) are sterilized during said step of sterilizing.
